# EUROPEAN PATENT SPECIFICATION

(11) **EP 1 197 181 B1**
(45) Date of publication and mention of the grant of the patent: **17.11.2004**
(21) Application number: 01128856.0
(22) Date of filing: 28.03.1995
(51) Int. Cl.: A61B 17/064

(54) **Apparatus for spinal fixation**
Einrichtung zur Wirbelsäulenfixation
Appareil de fixation vertébrale

(30) Priority: 28.03.1994 US 219626
(43) Date of publication of application: 17.04.2002
(62) Divisional of application: 95914197.9
(73) Proprietor: MICHELSON, Gary Karlin, Venice, CA 90291 (US)
(72) Inventor: MICHELSON, Gary Karlin, Venice, CA 90291 (US)
(74) Representative: Viering, Jentschura & Partner

(56) References cited:
- DE-B- 2 649 042
- ES-A- 2 048 671
- US-A- 4 401 112
- US-A- 5 015 247
- US-A- 5 092 893

## Description

This invention relates to surgical interbody fixation devices and in particular to a surgically implantable device for the stabilization of adjacent vertebrae of the human spine undergoing spinal arthrodesis and for the prevention of the dislodgement of spinal fusion implants used in the fusion process.

### Description of the Related Art

When a segment of the human spine degenerates, or otherwise becomes diseased, it may become necessary to surgically remove the affected disc of that segment, and to replace it with bone for the purpose of obtaining a spinal fusion by which to restore more normal, pre-morbid, spatial relations, and to provide for enhanced stability across that segment. Performing such surgery of the spine from an anterior (front) approach offers the great advantage of avoiding the spinal cord, dural sac; and nerve roots. Unfortunately, in entering the disc space anteriorly a very important band-like structure called the anterior longitudinal ligament, is violated. This structure physiologically acts as a significant restraint resisting the anterior displacement of the disc itself and acting as a tension band binding the front portions of the vertebrae so as to limit spinal hyperextension.

Historically, various devices have been utilized in an attempt to compensate for the loss of this important stabilizing structure. These devices have assumed the form of blocks, bars, cables, or some combination thereof, and are bound to the vertebrae by screws, staples, bolts, or some combination thereof. The earliest teachings are of a metal plate attached to adjacent vertebrae with wood-type screws. Dwyer teaches the use of a staple-screw combination. Brantigan U.S. Patent No. 4,743,256 issued on May 10, 1988, teaches the use of a block inserted to replace the disc, affixed to a plate then screwed to the vertebrae above and below. Raezian U.S. Patent No. 4,401,112 issued on August 30, 1993, teaches the use of a turnbuckle affixed to an elongated staple such that at least one entire vertebral body is removed, the turnbuckle portion is placed within the spine, and the staple extends both above and below the turnbuckle and engages the adjacent vertebrae to the one removed.

Unfortunately, both staples and screws have quite predictably demonstrated the propensity to back out from the vertebrae. This is quite understandable as any motion, either micro or macro, tends to stress the interface of the metallic implant to the bone, and in doing so causes the bone to relieve the high stress upon it by resorbing and moving away from the metal. This entropic change is universally from the more tightened and thus well-fixated state, to the less tightened and less fixated state. For a staple, this is specifically from the more compressed and engaged state, to the less compressed and disengaged state. Similarly, screws in such a dynamic system loosen and back out.

The potential consequences of such loosening and consequent backing out of the hardware from the anterior aspect of the vertebral column may easily be catastrophic. Because of the proximity of the great vessels, aortic erosions and perforations of the vena cava and iliac vessels have usually occurred with unfortunate regularity and have usually resulted in death.

US 5,015,247 discloses an artificial spinal fusion implant comprising a cylindrical member having an outer diameter larger than the space between two adjacent vertebrae to be fused and a series of threads on the exterior of the cylindrical member for engaging said vertebrae to maintain the implant in place when placed between two adjacent vertebrae.

DE-A-26 49 042 discloses a correction implant comprising bone screws for screwing into the vertebral body, the screws having an open slot within the screw head, and comprising a compression rod which can be inserted in the screw head slots and which can be tensioned on both sides of the respective screw head, and comprising pressure distributing plates which can be placed under the screw heads and which are adapted to the shape of the vertebral bodies.

Therefore, the need exists for a device which is effective in restoring stability to a segment of the spine such as, but not limited to, the anterior aspect of the human spine and which will without danger remain . permanently fixated once applied.

### SUMMARY OF THE INVENTION

It is an object of the invention to provide an apparatus by which stability of a spine's segment can be effectively restored and which will safely and permanently be fixated once applied.

This is achieved by a multi-segment spinal alignment apparatus having the features of claim 1. Further advantageous embodiments of the invention are described in the dependent claims.

The invention is broadly applicable to the anterior, posterior and lateral aspects of the spinal column, be it the cervical, thoracic or lumbar area. In particular, the use of a staple member spanning the disc space and engaging the adjacent vertebrae which is applied to the anterior aspect of the spine is of great utility in restraining those vertebral bodies from moving apart as the spine is extended and thus is effective in replacing the anterior longitudinal ligament of the patient.

The spinal fixation device of the present invention may be coated with materials to promote bone fusion and thus promote the incorporation and ultimate entombment of the spinal fixation device into the bone fusion mass. The use of a bone fusion promoting material results in a speedier vertebra to vertebra fusion as bone may grow along the coated spinal fixation device bridging the two vertebrae so that the spinal fixation device acts as a trellis and supplies essential chemical elements to facilitate the bone fusion process.

A spinal fixation device may be used as an anchor such that a multiplicity of spinal fixation devices may then be interconnected via a cable, rod, bar, or plate, so as to achieve or maintain a multi-segmental spinal alignment.

The spinal fixation device could be made of resorbable materials, such as bio-compatible resorbable plastics, that resorb at an appropriate rate such that once the spinal fixation device is no longer needed (i.e. when spinal fusion is complete) the body would resorb the spinal fixation device. The spinal fixation device could be only in part resorbable such that the projections of the staple member would be non-resorbable and would remain incarcerated in the vertebrae and sealed off once the resorbable portion of the staple is resorbed by the body.

As a further alternative, the spinal fixation device could be made wholly of in part of ceramic and more particularly made of or coated with a ceramic such as hydroxyapatite that would actively participate in the fusion process.

It is another object of the present invention to provide a spinal fixation device that serves as an anchor, such that a multiplicity of these anchors may then be interconnected via a cable, rod, bar, or plate, so as to achieve or maintain a multi-segmental spinal alignment.

The invention will become further apparent from a review of the accompanying drawings and the detailed description of the drawings.

### BRIEF DESCRIPTION OF THE DRAWINGS

Figure 1 is a cross sectional view of a spinal fixation device of the present invention engaging two adjacent vertebrae and being attached to a spinal fusion implant, shown being used as an anchor for a multi-segmental spinal alignment means.
Figure 2 is an enlarged elevational side view of a threaded post used to connect the spinal fixation device of the present invention to a multi-segmental spinal alignment means.

### DETAILED DESCRIPTION OF THE DRAWINGS

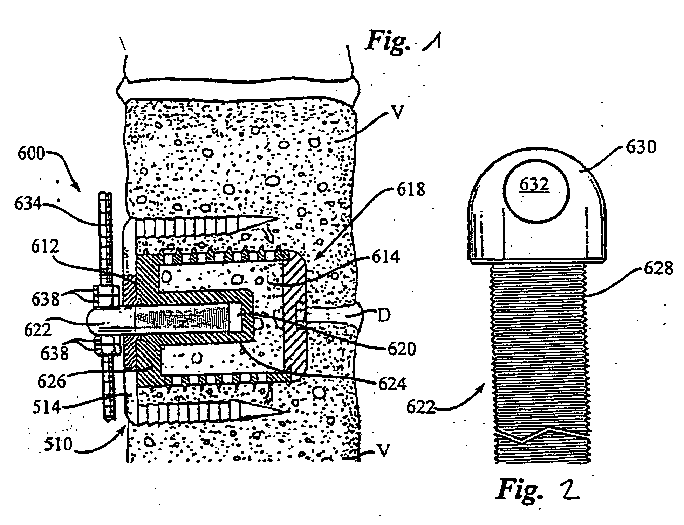

Referring to Figure 1 and 2, as an application, the spinal fixation device 510 of the present invention may be used as an anchor for a multi-segmental spinal alignment means 600, such that a multiplicity of spinal fixation devices may then be interconnected via a cable, rod, bar, or plate, so as to achieve or maintain any desired multi-segment spinal alignment. In the preferred embodiment, the multi-segmental spinal alignment means 600 comprises more than one spinal fixation device 510 of the present invention placed in series along the spine such that each spinal fixation device 510 spans one disc D and engages two adjacent vertebrae V. The spinal fixation device 510 is preferred over the other embodiments of the present invention in that it has a greater area of engagement with the vertebrae V so as to provide a solid anchoring means for the multi-segmental spinal alignment means 600. However, it is appreciated that other embodiments including but not limited to those described therein may be utilized as anchoring means for the multi-segmental spinal alignment means 600.

When used as an anchor, each spinal fixation device 510 interdigitates with and is connected to a spinal fusion implant 610 having an insertion end 612, an interior chamber 614 and is inserted in the disc space between the two adjacent vertebrae. The spinal fusion implant 610 has a threaded blind hole 620 for receiving a threaded post 622 therein. The blind hole 620 has a casing that is made of strong surgically, implantable material such as, but not limited to titanium. The casing 624 extends from the insertion end 612 of the spinal fusion implant 610 into the interior central chamber 614. The insertion end 612 has a rigid construction that is capable of withstanding high torsion forces resulting from the tensioning of the multi-segmental spinal alignment means to align segments of the spine. In the preferred embodiment, the insertion end 612 of the spinal fusion implant has an end portion 626 that closes the insertion end 612. The end portion is substantially thicker than the rest of the spinal fusion implant 610 and in the preferred embodiment, the end portion 626 has thickness ranging from 1.5 mm to 4.0 mm, with 2.5 mm being the preferred thickness.

Referring to Figure 2, the threaded post 622 has a threaded end 628 with a locking thread pattern that is substantially longer than the locking thread pattern 62 of the screw 60 described above and a head portion 630 having a hole 632 for receiving a rod 634 or a cable therethrough. The head portion 630 has a rounded exterior surface to prevent any damage such as aortic erosion to the vessels in the area adjacent to the spine. In the preferred embodiment the threaded post has a diameter ranging from 3.0 mm to 6.0 mm, with 4.5 mm being the preferred diameter and has a length ranging from 15.0 mm to 25.0 mm, with 20.0 mm being the preferred length. The head portion 630 extends at a height above the top member 514 of the spinal fixation device 510 of approximately 8.0 mm to 16.0 mm, with 12.0 being the height preferred once it is threadably attached to the spinal fusion implant 610 such that it does not significantly protrude from the spinal column into the tissue and vessels adjacent thereto.

Once the threaded post 622 is attached to'the spinal fusion implant 610, the head portion 630 of each threaded post 622 are connected to one another by the rod 634 having a sufficient diameter to fit through the hole 632 of each head portion 630. The rod 634 has at least a portion thereof that is threaded so that a plurality of lock nuts 638 may be used to secure the rod 634 to the head portions 630. The lock nuts 638 may also be used as length adjusting means to adjust the length of the rod 634 between head portions 630 so that segmental portions of the spine may be held closer together or held further aport for the purposes of aligning the spine. It is appreciated that a plurality of multi-segmental spinal alignment means 600 may be placed in series either on one side or on opposite sides of the spine, such that one side of the spine may be extended while the other side may be held stationary or may be compressed in order to achieve proper spinal alignment. The multi-segment spinal alignment may be maintained by keeping the rod tensioned with the lock nuts 638 or by any other means well known by those skilled in the art. It is also appreciated that in place of a rod 634 a cable, a plate or any other means well known by those skilled in the art may be used to interconnect the multi-segmental spinal alignment means.

## Claims

1. A multi-segmental spinal alignment apparatus for linking segments of the spine, comprising:
a first spinal implant (610) adapted to be surgically implanted at least in part within a first disc space between two adjacent vertebrae in a segment of the spine, said first spinal implant (610) being adapted to contact both of the vertebrae adjacent to the first disc space when the disc space has been restored to approximate a normal height for the disc space;
a second spinal implant (610) adapted to be surgically implanted at least in part within a second disc space between two adjacent vertebrae in another segment of the spine, said second spinal implant (610) being adapted to contact both of the vertebrae adjacent to the second disc space when the disc space has been restored to approximate a normal height for the disc space; and
a connector (634) attached to said first and second spinal implants (610) for connecting said first and second spinal implants (610).

2. The apparatus of claim 1, wherein said first and second spinal implants (610) have an end portion configured to receive said connector (634).

3. The apparatus of claim 2, wherein each of said end portions is a coupler (622).

4. The apparatus of claim 3, wherein each of said couplers (622) are detachable.

5. The apparatus of either claim 3 or 4, wherein each of said couplers (622) includes an aperture (632) generally along the longitudinal axis of the spine for receiving said connector (634).

6. The apparatus of either claim 3 or 4, wherein each of said couplers (622) has a head (630) and a shank (628), said head having an aperture (632) for receiving the connector (634).

7. The apparatus of claim 6, wherein each of said first and second implants (610) has an aperture (620) for receiving said shanks (628).

8. The apparatus of claim 7, wherein said shanks (628) and said apertures (620) have cooperating threads.

9. The apparatus of any one of the above claims, wherein said first and second implants (610) include upper and lower portions with at least one opening in each of said upper and lower portions for permitting bone growth from one of the adjacent vertebrae through said spinal implant (610) to the other of the adjacent vertebrae.

10. The apparatus of claim 9, further comprising a hollow interior (614) within each of said first and second implants (610) for holding bone growth promoting material, said hollow interior (614) being in communication with at least one opening in each of said upper an lower portions.

11. The apparatus of any one of the above claims, wherein said first and second implants (610) include upper and lower portions that are arcuate.

12. The apparatus of any one of the above claims, wherein said first and second implants (610) further comprise a protrusion for engaging the adjacent vertebrae.

13. The apparatus of claim 12, wherein said protrusion is a thread.

14. The apparatus of any one of the above claims, wherein said connector (634) is selected from one of a rod, a cable, a plate, and a bar.

15. The apparatus of claim 1, further comprising means for adjusting the length of said connector (634) between said first and second spinal implants (610) for aligning segments of the spine.

16. The apparatus of claim 15, wherein said length adjusting means includes a threaded member an said connector (634) for varying the length of said connector (634) between said first and second spinal implants (610).

17. The apparatus of either claim 15 or 16, further comprising a lock (638) for locking said length adjusting means at a selected length.

18. The apparatus of any one of claims 1-17, in combination with a fusion promoting material to facilitate the bone fusion process.

19. The combination of claim 18, wherein said fusion promoting material includes at least one of hydroxyapatite, hydroxyapatite tricalcium phosphate, and bone morphogenic protein.

## Patentansprüche

1. Mehrfach-Segment-Wirbelsäulenausrichtungsvorrichtung zum Verbinden von Segmenten der Wirbelsäule, aufweisend:
ein erstes Wirbelsäulenimplantat (610), das angepasst ist, zumindest teilweise in einen ersten Bandscheibenraum zwischen zwei benachbarten Wirbelkörpern in einem Segment der Wirbelsäule chirurgisch implantiert zu werden, wobei das erste Wirbelsäulenimplantat (610) angepasst ist, beide zu dem ersten Bandscheibenraum benachbarten Wirbelkörper zu berühren, wenn der Bandscheibenraum wieder hergestellt wurde 'und eine für einen Bandscheibenraum normale Höhe angenommen hat,
ein zweites Wirbelsäulenimplantat (610), das angepasst ist, zumindest teilweise in einen zweiten Bandscheibenraum zwischen zwei benachbarten Wirbelkörpern in einem anderen Segment der Wirbelsäule chirurgisch implantiert zu werden, wobei das zweite Wirbelsäulenimplantat (610) angepasst ist, beide zu dem zweiten Bandscheibenraum benachbarte Wirbelkörper zu berühren, wenn der Bandscheibenraum wieder hergestellt wurde und eine für einen Bandscheibenraum normale Höhe angenommen hat, und
einen Verbinder (634), der an dem ersten und dem zweiten Wirbelsäulenimplantat (610) zum Verbinden des ersten mit dem zweiten Wirbelsäulenimplantat (610) angebracht ist.

2. Vorrichtung nach Anspruch 1, wobei das erste und das zweite Wirbelsäulenimplantat (610) einen Endbereich aufweisen, der konfiguriert ist, den Verbinder (634) aufzunehmen.

3. Vorrichtung nach Anspruch 2, wobei jeder der Endbereiche ein Kuppler (622) ist.

4. Vorrichtung nach Anspruch 3, wobei jeder der Kuppler (622) abnehmbar ist.

5. Vorrichtung nach Anspruch 3 oder 4, wobei jeder der Kuppler (622) im allgemeinen entlang der Längsachse der Wirbelsäule eine Aussparung (632) zum Aufnehmen des Verbinders (634) aufweist.

6. Vorrichtung nach Anspruch 3 oder 4, wobei jeder der Kuppler (622) einen Kopf (630) und einen Schaft (628) aufweist, wobei der Kopf eine Aussparung (632) zum Aufnehmen des Verbinders (634) aufweist.

7. Vorrichtung nach Anspruch 6, wobei jedes des ersten und des zweiten Implantats (610) eine Aussparung (620) zum Aufnehmen eines Schaftes (628) aufweist.

8. Vorrichtung nach Anspruch 7, wobei die Schäfte (628) und die Aussparungen (620) jeweils zusammenwirkende Gewinde aufweisen.

9. Vorrichtung nach einem der obigen Ansprüche, wobei das erste und das zweite Implantat (610) einen oberen und einen unteren Bereich mit zumindest einer Öffnung in jedem von dem oberen und dem unteren Bereich zum Ermöglichen von Knochenwachstum von einem der benachbarten Wirbelkörper durch das Wirbelsäulenimplantat (610) hindurch zu dem anderen der benachbarten Wirbelkörper aufweist.

10. Vorrichtung nach Anspruch 9, die ferner ein hohles Inneres (614) innerhalb jedes von dem ersten und zweiten Implantat (610) zum Halten von Knochenwachstums-Förderungsmaterial aufweist, wobei das hohle Innere (614) in Kommunikation mit zumindest einer Öffnung in jedem von dem oberen und dem unteren Bereich ist.

11. Vorrichtung nach einem der obigen Ansprüche, wobei das erste und das zweite Implantat (610) einen oberen und einen unteren Bereich aufweisen, die bogenförmig sind.

12. Vorrichtung nach einem der obigen Ansprüche, wobei das erste und das zweite Implantat (610) ferner einen Vorsprung zum Eingriff in den benachbarten Wirbelkörper aufweisen.

13. Vorrichtung nach Anspruch 12, wobei der Vorspruch ein Gewinde ist.

14. Vorrichtung nach einem der obigen Ansprüche, wobei der Verbinder (634) aus einem Stab, einem Seil, einer Platte bzw. einem Balken ausgewählt ist.

15. Vorrichtung nach Anspruch 1, die ferner ein Mittel zum Einstellen der Länge des Verbinders (634) zwischen dem ersten und dem zweiten Wirbelsäulenimplantat (610) zum Ausrichten der Segmente der Wirbelsäule aufweist.

16. Vorrichtung nach Anspruch 15, wobei das Längen-Ausrichtungsmittel ein Gewindeelement an dem Verbinder (634) zum Variieren der Länge des Verbinders (634) zwischen dem ersten und dem zweiten Wirbelsäulenimplantat (610) aufweist.

17. Vorrichtung nach Anspruch 15 oder 16, die ferner eine Verriegelungsvorrichtung (638) zum Verriegeln des Längen-Ausrichtungsmittels bei einer gewünschten Länge aufweist.

18. Vorrichtung nach einem der Ansprüche 1-17 in Kombination mit einem Fusions-Fördermaterial, um den Knochenfusionsprozess zu ermöglichen.

19. Kombination nach Anspruch 18, wobei das Fusion-Fördermaterial zumindest eines von Hydroxyapatit, Hydroxyapatit-Tricalziumphosphat oder Knochenmorphogenetisches Protein aufweist.

## Revendications

1. Appareil d'alignement spinal multi-segments pour relier des segments de la colonne vertébrale, comprenant :
un premier implant spinal (610) adapté pour être implanté chirurgicalement au moins en partie dans un premier espace discal entre deux vertèbres adjacentes dans un segment de la colonne vertébrale, ledit premier implant spinal (610) étant adapté pour être en contact avec les deux vertèbres adjacentes au premier espace discal lorsque l'espace discal a été rétabli pour atteindre approximativement une hauteur normale pour l'espace discal ;
un second implant spinal (610) adapté pour être implanté chirurgicalement au moins en partie dans un second espace discal entre deux vertèbres adjacentes dans un autre segment de la colonne vertébrale, ledit second implant spinal (610) étant adapté pour être en contact avec les deux vertèbres adjacentes au second espace discal lorsque l'espace discal a été rétabli pour atteindre approximativement une hauteur normale pour l'espace discal ; et
un dispositif de connexion (634) fixé aux dits premier et second implants spinaux (610) pour raccorder lesdits premier et second implants spinaux (610).

2. Appareil selon la revendication 1, dans lequel lesdits premier et second implants spinaux (610) ont une partie terminale configurée pour recevoir ledit dispositif de connexion (634).

3. Appareil selon la revendication 2, dans lequel chacune desdites parties terminales est un dispositif de couplage (622).

4. Appareil selon la revendication 3, dans lequel chacun des dits dispositifs de couplage (622) est détachable.

5. Appareil selon la revendication 3 ou 4, dans lequel chacun des dits dispositifs de couplage (622) inclut une ouverture (632) généralement le long de l'axe longitudinal de la colonne vertébrale pour recevoir ledit dispositif de connexion (634).

6. Appareil selon la revendication 3 ou 4, dans lequel chacun des dits dispositifs de couplage (622) a une tête (630) et une tige (628), ladite tête ayant une ouverture (632) pour recevoir le dispositif de connexion (634).

7. Appareil selon la revendication 6, dans lequel chacun desdits premier et second implants (610) a une ouverture (620) pour recevoir lesdites tiges (628).

8. Appareil selon la revendication 7, dans lequel lesdites tiges (628) et les dites ouvertures (620) ont des filetages coopératifs.

9. Appareil selon l'une quelconque des revendications précédentes, dans lequel lesdits premier et second implants (610) incluent des parties supérieure et inférieure avec au moins une ouverture dans chacune desdites parties supérieure et inférieure pour permettre la croissance de l'os depuis l'une des vertèbres adjacentes à travers ledit implant spinal (610) vers l'autre des vertèbres adjacentes.

10. Appareil selon la revendication 9, comprenant en outre un intérieur creux (614) dans chacun desdits premier et second implants (610) pour contenir un matériau favorisant la croissance osseuse, ledit intérieur creux (614) étant en communication avec au moins une ouverture dans chacune desdites parties supérieure et inférieure.

11. Appareil selon l'une quelconque des revendications précédentes, dans lequel lesdits premier et second implants (610) incluent des parties supérieure et inférieure arquées.

12. Appareil selon l'une quelconque des revendications précédentes, dans lequel lesdits premier et second implants (610) comprennent en outre une protubérance pour engager les vertèbres adjacentes.

13. Appareil selon la revendication 12, dans lequel ladite protubérance est un filetage.

14. Appareil selon l'une quelconque des revendications précédentes, dans lequel ledit dispositif de connexion (634) est choisi parmi une tige, un câble, une plaque et une barre.

15. Appareil selon la revendication 1, comprenant en outre un moyen pour ajuster la longueur du dit dispositif de connexion (634) entre lesdits premier et second implants spinaux (610) pour aligner des segments de la colonne vertébrale.

16. Appareil selon la revendication 15, dans lequel ledit moyen d'ajustement de la longueur inclut un membre fileté sur ledit dispositif de connexion (634) pour faire varier la longueur du dit dispositif de connexion (634) entre lesdits premier et second implants spinaux (610).

17. Appareil selon la revendication 15 ou 16, comprenant en outre un verrou (638) pour verrouiller ledit moyen d'ajustement de la longueur à une longueur sélectionnée.

18. Appareil selon l'une quelconque des revendications 1 à 17, en association avec un matériau favorisant la fusion pour faciliter le processus de fusion osseuse.

19. Appareil selon la revendication 18, dans lequel ledit matériau favorisant la fusion inclut au moins un parmi l'hydroxyapatite, le phosphate d'hydroxyapatite tricalcium une protéine morphogène de l'os.
